# EUROPÄISCHE PATENTSCHRIFT

(11) **EP 2 384 725 B1**
(45) Veröffentlichungstag und Bekanntmachung des Hinweises auf die Patenterteilung: **04.07.2018**
(21) Anmeldenummer: 11162224.7
(22) Anmeldetag: 13.04.2011
(51) Int. Cl.: A61F 2/82, A61L 31/14

(54) **Biokorrodierbares Implantat, bei dem eine Korrosion nach erfolgter Implantation durch einen externen Stimulus ausgelöst oder beschleunigt werden kann**
Biocorrodable implant in which corrosion may be triggered or accelerated after implantation by means of an external stimulus
Implant biocorrodable, dans lequel une corrosion peut être déclenchée et accélérée une fois l'implantation effectuée par un stimulus externe

(30) Priorität: 06.05.2010 US 331868 P
(43) Veröffentlichungstag der Anmeldung: 09.11.2011
(73) Patentinhaber: Biotronik AG, 8180 Bülach (CH)
(72) Erfinder: Dörr, Thomas, 12437 Berlin (DE); Borck, Alexander, 91086 Aurachtal (DE)
(74) Vertreter: Galander, Marcus

(56) Entgegenhaltungen:
- WO-A2-2006/108065
- WO-A2-2008/036554
- US-A1- 2007 141 106
- US-A1- 2008 199 510

## Beschreibung

Die Erfindung betrifft ein biokorrodierbares Implantat wie eine Gefäßstütze oder ein orthopädisches Implantat.

Implantate haben in vielfältiger Ausführungsform Anwendung in der modernen Medizintechnik gefunden. Sie dienen beispielsweise der Unterstützung und / oder Stützung von Gefäßen, Hohlorganen und Gangsystemen (Endovaskuläre Implantate, z. B. Stents), zur Befestigung und temporären Fixierung von Gewebeimplantaten und Gewebstransplantationen, aber auch zu orthopädischen Zwecken, zum Beispiel als Nagel, Platte oder Schraube. Häufig ist nur eine temporäre Stütz- beziehungsweise Haltefunktion bis zum Abschluss des Heilungsprozesses oder Stabilisierung des Gewebes notwendig beziehungsweise erwünscht. Um Komplikationen zu vermeiden, die aus dem dauerhaften Verbleib der Implantate im Körper resultieren, müssen die Implantate entweder wieder operativ entfernt werden oder sie bestehen aus einem Werkstoff, der allmählich im Körper abgebaut wird, das heißt biokorrodierbar ist. Die Zahl biokorrodierbarer Werkstoffe auf der Basis von Polymeren oder Legierungen ist stetig gewachsen. So sind unter anderem biokorrodierbare Metalllegierungen der Elemente Magnesium, Eisen und Wolfram bekannt. Eine besonders häufig verwendete Form eines Implantats ist der Stent.

Die Implantation von Gefäßstützen wie beispielsweise Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch bildgebende Verfahren, wie z. B. durch Röntgenuntersuchungen erfolgen.

Der Stent besitzt einen Grundkörper aus einem Implantatswerkstoff. Ein solcher Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßer Verwendung mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe für Stents umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder Ti-Al6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Kalzium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest <1% bekannt, die sich insbesondere zur Herstellung einer Endoprothese, zum Beispiel in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen. Solche biokorrodierbaren Implantate und Stents weisen häufig auch eine Beschichtung oder Kavitätenfüllung mit einem geeigneten Polymer auf. Auch bekannt sind Stents aus biokorridierbarern Polymeren wie Polylactid (PLA), Poly-L-Lactid (PLLA), Poly-D-Lactid (PDLA), Polyglycolid (PGA), Polydioxanon, Polycaprolacton, Polygluconat, Polymilchsäure-Polyethylenoxid-Kopolymere, modifizierter Zellulose, Kollagen, Polyhydroxybutyrat (Polyhydroxybuttersäure/PHB), Polyanhydrid, Polyphosphoester, Polyaminosäuren, Poly(alphahydroxysäure) oder verwandten Kopolymerwerkstoffen.

Ein Problem biokorrodierbarer Implantate, insbesondere biokorrodierbarer Stents, ist die Schwierigkeit eine Standzeit zu gewährleisten, die lang genug ist, um die gewünschte medizinische Wirkung zu erreichen. Für gewöhnlich beginnt das Implantat bereits unmittelbar nach der Implantation zu korrodieren. Zwar sind Beschichtungen, bevorzugt selbst biokorrodierbare Beschichtungen, bekannt, die einen solchen Biokorrosionsprozess verlangsamen können. Allerdings setzt auch bei einem verlangsamten Biokorrosionsprozess der Verlust an Stabilität bzw. der Integrität des Implantats bereits kurz nach der Implantation ein, so dass das Implantat über die Zeit zunehmend instabiler wird. Zwar wird im Dokument WO 2006/108065 ein biokorrodierbares Implantat offenbart, bei dem eine Korrosion nach erfolgter Implantation durch einen externen Stimulus ausgelöst oder beschleunigt werden kann, wobei das Implantat einen Grundkörper aufweist, der ganz oder in Teilen einen biokorrodierbaren metallischen Werkstoff umfasst oder daraus besteht, und der Grundkörper eine Passivierungsschicht wie beispielsweise eine Oxidschicht aufweist, die nicht biokorrodierbar ist. Im genannten Dokument wird die die Korrosion dadurch auslöst oder beschleunigt, dass die Passivierungsschicht gezielt durch mechanische Stimuli (durch Rotablatoren, Cutting balloons oder dergleichen) zerstört wird. Dies bedeutet aber eine längere Operationsprozedur oder weitere Eingriffe in den Organismus, was vermieden werden soll. Ansonsten ist es derzeit nicht möglich, die Stabilität bzw. Integrität eines biokorrodierbaren Implantats über die gewünschte Standzeit im Wesentlichen ohne Beeinträchtigung zu gewährleisten und einen Biokorrosionsprozess des Implantats erst nach Ablauf der gewünschten Standzeit in nennenswertem Umfang einsetzen zu lassen. Ein Implantat, das nach einen externen Stimulus korrodiert, ist aus US 2008/199510 A1 vorbekannt. Aufgabe der vorliegenden Erfindung gemäss Anspruch 1 ist es mindestens einen der Nachteile des Standes der Technik zu vermindern oder zu vermeiden.

Diese Aufgabe wird gelöst durch Bereitstellung eines biokorrodierbaren Implantats bei dem eine Korrosion nach erfolgter Implantation durch einen externen Stimulus ausgelöst oder beschleunigt werden kann, wobei das Implantat einen Grundkörper aufweist, der ganz oder in Teilen aus einem biokorrodierbaren metallischen Werkstoff besteht, und der Grundkörper eine Beschichtung mit einer Schutzschicht aufweist, die nicht biokorrodierbar ist, dadurch gekennzeichnet, dass das Implantat Regelelemente aufweist, die derart angeordnet und ausgebildet sind, so dass die Schutzschicht gegebenenfalls in Kombination mit den Regelelementen den Grundkörper ganz oder in Teilen undurchlässig für Körpermedium umschließt, wobei die Schutzschicht durch eine, durch einen externen Stimulus regel- und/oder steuerbare, Formänderung der Regelelemente in eine für Körpermedium durchlässige Form überführbar ist.Im Folgenden und im Sinne der Erfindung werden als Körpermedium alle im Körper natürlich oder unnatürlich vorhandene Medien bezeichnet. Dazu gehören Flüssigkeiten, die Wasser enthalten, wie beispielsweise Blut, Lymphflüssigkeit, Speichel, Wasserstoff, Sauerstoff, Stickstoff, Kohlendioxid, Ionen und Ionenlösungen enthaltend beispielsweise Phosphat oder Kalzium. Das erfindungsgemäße Implantat zeichnet sich dadurch aus, dass das Implantat durch die Schutzschicht nach erfolgter Implantation vor einer unkontrollierten Biokorrosion bewahrt wird. Erst durch Gabe eines externen Stimulus, beispielsweise durch Zufuhr einer externen Energie z. B. von einer externen (ex vivo) Quelle, wird der Grundkörper des Implantats zugänglich und einer Korrosion zugeführt. Durch Kontrolle des externen Stimulus kann also das Korrosionsverhalten des erfindungsgemäßen Implantats nach erfolgter Implantation von extern geregelt und/oder gesteuert werden. Dazu weist das Implantat Regelelemente auf, die, ausgelöst durch einen externen Stimulus, eine vorher definierte Formänderung durchlaufen können. Diese Regelelemente sind derart ausgebildet und angeordnet, so dass das Implantat zunächst von der Schutzschicht, ggf. in Kombination mit den Regelelementen, für Körpermedium undurchlässig umschlossen vorliegt. Wird nun durch Applikation eines externen Stimulus eine Formänderung der Regelelemente hervorgerufen, so wird der Grundkörper des Implantats nicht länger dicht für Körpermedium abgeschlossen, sondern ist für das Körpermedium zugänglich geworden, so dass nun ein Biokorrosionsprozess beginnen oder beschleunigt werden kann.

Bevorzugt ist das erfindungsgemäße Implantat ein Stent.

Das erfindungsgemäße Implantat weist einen Grundkörper auf, der ganz oder in Teilen aus einem biokorrodierbaren Werkstoff gebildet ist. Vorzugsweise weist das Implantat einen Grundkörper auf, der ganz oder in Teilen aus einem biokorrodierbaren, metallischen Werkstoff besteht. Insbesondere kann der biokorrodierbare, metallische Werkstoff Magnesium, eine biokorrodierbare Magnesiumlegierung, Reineisen, eine biokorrodierbare Eisenlegierung, eine biokorrodierbare Wolframlegierung, eine biokorrodierbare Zinklegierung oder eine biokorrodierbare Molybdänlegierung aufweisen oder daraus bestehen.

Als biokorrodierbar im Sinne der Erfindung werden Stoffe, Werkstoffe, Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Die Legierung ist so in ihrer Zusammensetzung zu wählen, dass sie biokorrodierbar ist. Besonders geeignete biokorrodierbare Magnesiumlegierungen sind in DE 102 53 634 A1 offenbart und umfassen eine biokorrodierbare Magnesiumlegierung mit einem Anteil von Magnesium >90 %, Yttrium 3,7 - 5,5 %, Seltenerdmetallen 1,5 - 4,4 % und Rest <1%. Diese Magnesiumlegierung eignet sich besonders gut zur Herstellung einer Endoprothese, zum Beispiel in Form eines selbstexpandierenden oder ballonexpandierbaren Stents.

Als Prüfinedium zur Testung des Korrosionsverhaltens eines in Frage kommenden Werkstoffs dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Werkstoffe wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen. Ein Stoff wird beispielsweise dann als nicht biokorrodierbar bezeichnet, wenn der Stoff in oben genanntem Test nach Ablauf von 12 Monaten zu nicht mehr als 50% korrodiert oder umgesetzt ist.

Eine Schutzschicht im Sinne der Erfindung ist eine zumindest abschnittsweise auf den Grundkörper des Implantats aufgetragene Beschichtung. Vorzugsweise wird die gesamte Oberfläche des Grundkörpers des Implantats von der Beschichtung bedeckt. Eine Schichtdicke der Schutzschicht liegt vorzugsweise im Bereich von 1 nm bis 100 µm, besonders bevorzugt 300 nm bis 15 µm. Die Schutzschicht kann direkt auf die Implantatoberfläche aufgetragen werden, es können aber auch eine oder mehrere zusätzliche Schichten zwischen der Implantatoberfläche und der Schutzschicht vorgesehen sein. Bei einer solchen zusätzlichen Schicht kann es sich beispielsweise um eine die Haftung der Schutzschicht verbessernde Haftschicht handeln. Die Verarbeitung kann nach Standardverfahren für die Beschichtung erfolgen. Es können einschichtige, aber auch mehrschichtige Systeme erstellt werden. Insbesondere kann das erfindungsgemäße Implantat, dadurch gekennzeichnet, dass zwischen dem Grundkörper und der Schutzschicht eine zusätzliche Schicht vorgesehen ist, die die Haftung der Schutzschicht verbessert.

Die Schutzschicht ist nicht biokorrodierbar und kann eine Vielzahl bekannter Materialien und Polymeren umfassen oder daraus bestehen. Beispielsweise kann die Schutzschicht Poly-L-Lactid enthalten oder daraus bestehen oder einen weiteren Vertreter der Polyester wie PDLLA, PLGA, P3HB, P4HB oder Gemische oder Copolymere daraus. Alternativ oder zusätzlich kann die Schutzschicht Parylen aufweisen (Parylen C oder andere Derivate), bevorzugt als Parylen mit sog. "pin holes". Daneben oder stattdessen kann die Schutzschicht Cellulose aufweisen, bevorzugt als Film, wie beispielsweise Nitrocellulose, Methylcellulose, Carboxymethylcellulose. Die Schutzschicht kann auch Polyvinylalkohole aufweisen, wobei durch Wahl der Molmasse und des Deacetylierungsgrades eine Filmbildung optimiert werden kann. Polyvinylalkohol ist ein kristallines Polymer, das aufgrund seines Herstellungsverfahrens geringfügig verzweigt ist. Polyvinylacetat wird aus Vinylacetat hergestellt.Das Polyvinylacetat wird durch Umsatz mit Basen zum Polyvinylalkohol hydroliesiert. Die Schmelz- und Glasübergangstemperatur hängt außer vom Hydrolysegrad und der Molmasse auch von der Verteilung der Acetylgruppen (statistisch oder in Blöcken) der Taktizität und dem Wassergehalt des Polymers ab. Geeignet sind Polyvinylalkohole die hohe bis mittlere Hydrolysierungsgrade aufweisen und Polymerisationsgrade bis 2000 besitzen. Die aus Polyvinylalkohol hergestellten Filme sind reißfest und zähelastisch. Sie sind öl- und hitzebeständig. Als Weichmacher können Polyalkohole, wie z. B. Glycerin und Ethylenglycol, verwendet werden. Die Schutzschicht, ggf. in Kombination mit den Regelelementen, umschließt den Grundkörper des Implantats ganz oder in Teilen für Körpermedium undurchlässig, so dass die Teile des Grundkörpers, die umschlossen sind, nicht direkt mit Körpermedium kontaktierbar sind.

Dazu kann die Schutzschicht als nicht-perforierte, für Körpermedium undurchlässige Beschichtung vorliegen, die den Grundkörper des Implantats ganz oder in Teilen umgibt. In diesem Fall sind die Regelelemente bevorzugt auf, in oder unter der Schutzschicht angebracht, so dass die Integrität und Dichte gegen Kontakt mit Körpermedium der Schutzschicht durch eine Formänderung der Regelelemente beeinträchtigt und derart aufgelöst werden kann, dass danach ursprünglich mit der Schutzschicht umschlossene Teile des Grundgerüsts nun mit Körpermedium kontaktierbar sind.

In einer anderen Ausführungsform liegt die Schutzschicht als perforierte Beschichtung bzw. Membran vor, wobei in den Perforationsöffnungen Regelelemente angeordnet sind, so dass die Schutzschicht in Kombination mit den Regelelementen zunächst den Grundkörper des Implantats ganz oder in Teilen für Körpermedium undurchlässig umgibt. In diesem Fall sind die Regelelemente auf oder in der Schutzschicht angeordnet, so dass die Integrität und Dichtigkeit der Schutzschicht gegen Körpermedium durch eine Formänderung der Regelelemente beeinträchtigt und derart aufgelöst werden kann, dass danach ursprünglich für Körpermedium undurchlässig umschlossene Teile des Grundgerüsts nun mit Körpermedium kontaktierbar sind.

Das erfindungsgemäße Implantat ist derart ausgestaltet, dass eine Formänderung der Regelelemente durch einen externen Stimulus regel- und/oder steuerbar ist. Dabei kann der externe Stimulus direkt die Formänderung der Regelelemente bewirken oder derart auf das Implantat einwirken, so dass mittelbar ein Signal erzeugt wird, welches die Formänderung der Regelelemente auslöst oder bewirkt. Der externe Stimulusumfasst oder besteht aus einer Temperaturänderung, elektromagnetischer Strahlung, Hochfrequenzstrahlung (RF), einem Ultraschallsignal, ionisierender Strahlung einem magnetischen Feld und/oder Licht in diversen Spektralbereichen. Geeignete Quellen für die Generierung eines solchen externen Stimulus sind dem Fachmann bekannt. Solche Quellen können Vorrichtungen umfassen zur Applikation eines oder mehrerer der vorgenannten externen Stimuli, beispielsweise zur Applikation von Ultraschallenergie, bevorzugt von hochintensiver, fokussierter Ultraschallenergie (HIFU), zur Applikation von Hochfrequenzstrahlung oder von ionisierender Strahlung sowie von Magnetfeldern. Geeignete Magnetfeldern können von MRT-Geräten erzeugt und bereitgestellt werden.

In einer besonderen Ausführungsform ist das Implantat derart ausgebildet, so dass durch den externen Stimulus im Implantat eine Temperaturänderung hervorrufbar ist. Neben der direkten Applikation von Wärmeenergie, kann eine solche Temperaturänderung im Implantat beispielsweise durch Bestrahlen des Grundkörpers des Implantats mit einer Hochfrequenzstrahlung oder durch Anlegen eines Magnetfeldes erreicht werden.

Die Schutzschicht des Implantats kann Mittel enthalten, die eine Übersetzung eines externen Stimulus in eine Temperaturänderung erlauben. Dazu kann die Schutzschicht beispielsweise magnetische Partikel aufweisen, die durch Anlegen wechselnder geeigneter Magnetfelder in Schwingung versetzt werden können und somit eine Temperaturänderung im Implantat bewirken können.

Das erfindungsgemäße Implantat weist Regelelemente auf, die durch einen externen Stimulus regel- und/oder steuerbar eine Formänderung unterlaufen. Unter einer solchen Formänderung wird eine messbare Änderung des äußeren Erscheinungsbildes des Regelelementes verstanden, die sich nicht ausschließlich in der allgemeinen Wärmeausdehnung eines Körpers bei einer Temperaturerhöhung erschöpft. Solche Regelelemente enthalten oder bestehen bevorzugt aus Polymermaterialien, die die gewünschten Eigenschaften zeigen. Solche Polymermaterialien sind dem Fachmann bekannt. Bevorzugt werden Regelelemente eingesetzt, bei denen die Formänderung durch eine Temperaturänderung auslösbar ist.

In einer bevorzugten Ausführungsform enthält das Implantat Regelelemente die ein Hydrogel umfassen oder daraus bestehen. Ein Hydrogel ist ein Wasser enthaltendes, aber wasserunlösliches Polymer, dessen Moleküle chemisch, z. B. durch kovalente oder ionische Bindungen oder physikalisch, z. B. durch Verschlaufen der Polymerketten, zu einem dreidimensionalen Netzwerk verknüpft sind. Erfindungsgemäße Hydrogele sind in der Lage als Reaktion auf einen externen Stimulus ihr Volumen zu verändern, in dem sie ein veränderbares Quellvermögen aufweisen und damit regel- und/oder steuerbar unterschiedlich viel Wasser pro mmol Hydrogel-Polymer aufnehmen können. Diese Hydrogele können beispielsweise hergestellt werden durch Umsetzung ethylenisch ungesättigter Monomere und Polymere die ionisierbare Gruppen tragen mit Vernetzern und Polymerisationskatalysatoren. Alternativ dazu können geeignete Hydrogele hergestellt werden durch Kondensationsreaktionen mit difunktionellen und mehrfunktionellen Monomeren. Dem Fachmann sind geeignete Monomere und Polymere sowie Verfahren zu deren Herstellung bekannt. Ebenso sind dem Fachmann Methoden und Verfahren zur Herstellung von geeigneten Hydrogelen mittels solcher Mono- oder/und Polymere bekannt. Bevorzugte Hydrogele enthalten ein Polymer auf Basis von Acrylamid, Methacrylamid, Dimethylaminoethylmethacrylate oder einem Derivat von Acrylamid, Methacrylamid oder Dimethylaminoethylmethacrylate. Andere bevorzugte Hydrogele enthalten ein Polymer auf Basis von poly N-Isopropylacrylamide und/oder Poly-N-isopropylacrylamide-co-allylamin und/oder Poly-N-Isopropylamid (PNiPAM) oder Mischungen daraus mit Poly(p-dioxanon) als Hartsegment.

Unter dem Begriff "Quellvermögen" im Sinne der Erfindung wird die Eigenschaft des Hydrogels verstanden, eine bestimmte Wassermenge pro mmol Hydrogel-Polymer aufzunehmen. Ein verringertes Quellvermögen führt zu einer Verringerung des Volumens des Hydrogels und damit zu einer Formänderung des Hydrogels oder eines ein solches Hydrogel enthaltenden Regelelements. Geeignete Methoden und Messverfahren zur Bestimmung des Quellvermögens sind dem Fachmann bekannt, insbesondere eignen sich solche Messverfahren, wie sie sich im Bereich der Galenik bereits vielfach bewährt haben.

Bevorzugt werden Hydrogele eingesetzt, deren Quellvermögen temperaturabhängig ist. Besonders bevorzugte Hydrogele weisen bei steigenden Temperaturen ein verringertes Quellvermögen auf. Solche Hydrogele können sich dadurch auszeichnen, dass sich ihr Quellvermögen bei einer Temperaturerhöhung von 10K mindestens um 30% verringert, bevorzugt um bis zu 50%, besonders bevorzugt um 30% bis 50%.

Die Temperaturabhängigkeit des Hydrogels wird dabei bevorzugt so eingestellt, dass es bezüglich der Quelleigenschaften einen ausgeprägten Hystereseeffekt gibt, so dass das Quellvermögen auch bei Rückkehr zur Ausgangstemperatur von 37°C reduziert bleibt.

Durch die induzierte Erwärmung des Implantatsmaterials wird auch das Hydrogel erwärmt und dessen Quelleigenschaften so weit reduziert, dass die Formänderung des Hydrogels dazu führt, dass nun Körperflüssigkeiten das biodegradierbare Implantatsmaterial erreichen können und der Korrosionsprozess so gestartet wird.

In einer weiteren bevorzugten Ausführungsform weist das Implantat Regelelemente auf, die ein Formgedächtnis-Polymer umfassen oder daraus bestehen. Formgedächtnis-Polymere sind Kunststoffe, die einen sogenannten "shape memory"-Effekt aufweisen. Unter einem "shape memory"-Effekt wird dabei verstanden, dass ein Formgedächtnis-Polymer aus einer ursprünglichen Form in eine andere Form stabil überführt werden kann, in der es dann solange verbleibt, bis das Formgedächtnis-Polymer, als Reaktion auf einen externen Stimulus, zu einer vorherigen oder der ursprünglichen Form zurückkehrt. Weist das Implantat Regelelemente auf, die ein Formgedächtnis-Polymer umfassen, so kann das Formgedächtnis-Polymer als im Wesentlichen planar erstreckter Formkörper vorliegen, während der Fromkörper auf einen externen Stimulus hin beispielsweise in eine gebogene Form überführbar ist. Beim Übergang des Formgedächtnis-Polymers von der planaren zu der gebogenen Form kann die Schutzschicht perforiert werden und der Korrosionsprozess kann für das darunter liegende Grundgerüst des Implantats ausgelöst werden.

### Figuren:

- Figur 1:: zeigt den Ablauf der Biokorrosion eines Stents des Standes der Technik nach erfolgter Implantation.
- Figur 2:: zeigt den Ablauf der Biokorrosion eines erfindungsgemäßen Stents nach erfolgter Implantation.
- Figur 3:: zeigt eine schematische Darstellung einer ersten Ausführungsform eines erfindungsgemäßen Implantats.
- Figur 4:: zeigt eine schematische Darstellung einer zweiten Ausführungsform eines erfindungsgemäßen Implantats.
- Figur 5:: zeigt eine schematische Darstellung einer dritten Ausführungsform eines erfindungsgemäßen Implantats.

Nachfolgend wird die Erfindung anhand von Ausführungsbeispielen näher erläutert.

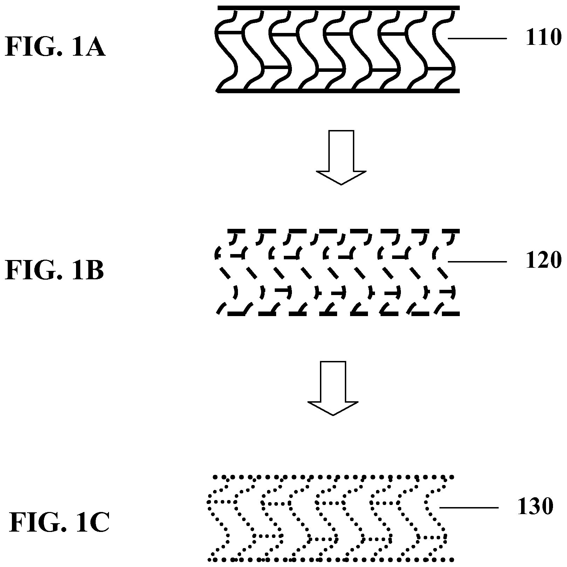

In Figur 1 ist der Ablauf einer Biokorrosion eines biokorrodierbaren medizinischen Implantates des Standes der Technik nach erfolgter Implantation am Beispiel eines Stents dargestellt. Wie in Fig. 1 (a) und (b) gezeigt, beginnt die Korrosion hier unmittelbar nach erfolgter Implantation 110, also schon zu Beginn der Standzeit, und verläuft entsprechend der ausgewählten Legierungseigenschaften im Wesentlichen kontinuierlich ab. Dadurch sind bereits in einem relativ frühen Stadium 120 die mechanischen Eigenschaften des Implantats deutlich verschlechtert (siehe Fig. 1 (b) und (c)), wobei der Grad an Verschlechterung auch von den individuellen Bedingungen im Gewebe des Empfängers abhängt. Im späteren Stadium (130) ist die erwünschte, nahezu vollständige Auflösung des Implantates dargestellt.

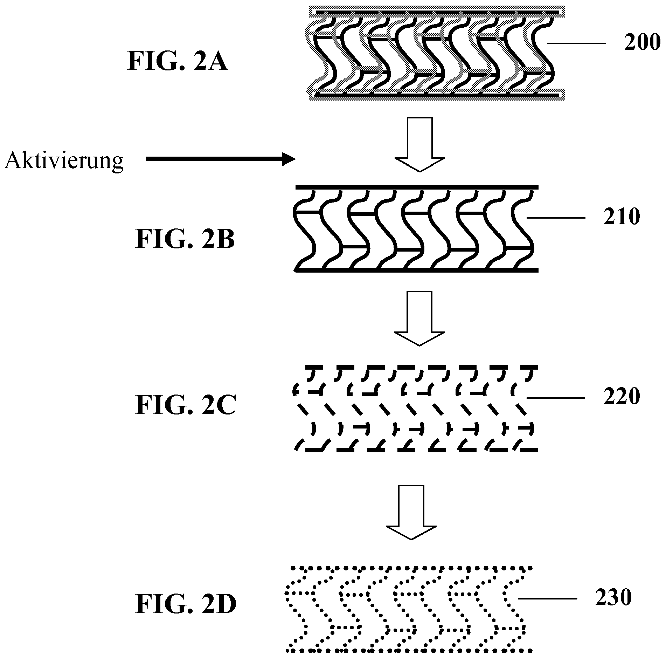

In Figur 2 (a) bis (d) hingegen ist der Ablauf einer Biokorrosion eines erfindungsgemäßen biokorrodierbaren Implantats am Beispiel eines Stents nach der Implantation dargestellt. Der Stent ist bei Implantation 200 mit einer nicht biodegradierbaren Schutzschicht (z. B. Polymer oder biokompatibles Wachs) überzogen. Erst wenn die Stützfunktion des Stents nicht mehr benötigt wird, wird diese Schutzschicht durch einen externen Stimulus perforiert (Aktivierung) und so der Prozess der Korrosion gestartet 210. Hier ist es nun möglich, für das Grundgerüst des Implantats Werkstoffe oder Materialkombinationen einzusetzen, die in relativ kurzer Zeit degradieren, wie z. B. Magnesiumlegierungen, so dass die Zeit zwischen dem Verlust der Stützfunktion 220 und einer vollständiger Auflösung 230 möglichst kurz gehalten werden kann.

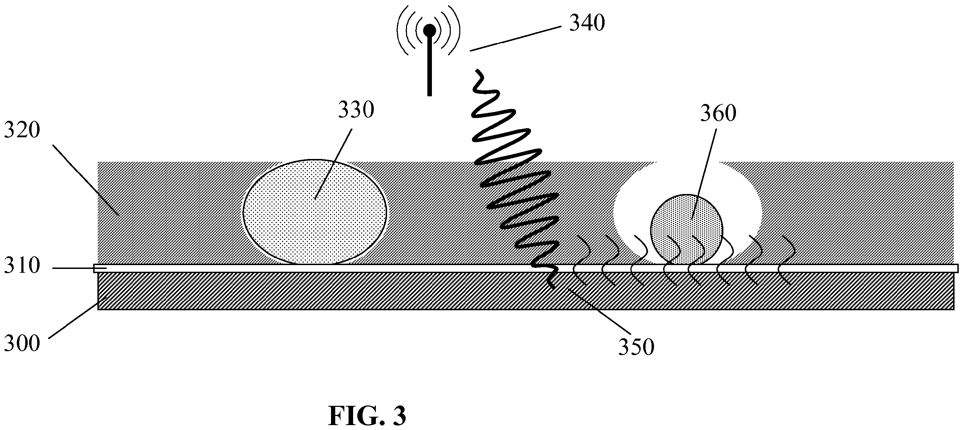

In Figur 3 ist ein erstes Ausführungsbeispiel eines erfindungsgemäßen Implantats schematisch dargestellt. Auf dem biodegradierbaren Material 300 des Implantates ist mittels eines Haftvermittlers 310 eine Schutzschicht in Form einer perforierten Membran 320 aufgebracht. Die Perforationsöffnungen der Membran 320 sind mit einem Hydrogel 330 verschlossen. Das eingesetzte Hydrogel 330 besitzt ein temperaturabhängiges Quellvermögen, das bei einer Erwärmung um 10K um etwa 30-50% sinkt. Die Temperaturabhängigkeit des Hydrogels 330 wird dabei bevorzugt so eingestellt, dass es bezüglich der Quelleigenschaften einen ausgeprägten Hystereseeffekt gibt, so dass die bewirkte Formänderung auch bei einer auf eine temporäre Erwärmung folgende Rückkehr zur Ausgangstemperatur von 37°C im Wesentlichen erhalten bleibt. Durch externe Einprägung von Hochfrequenz-Energie mittels eines Hochfrequenzsenders 340 kann eine temporäre Erwärmung des Implantates herbeigeführt werden. In dieser Anordnung ist die Wellenlänge des HochfrequenzSenders 340 auf die Antennengeometrie des biodegradierbaren Materials 300 des Implantates abgestimmt, so dass eine effektive Erwärmung möglich ist. Durch die induzierte Erwärmung des Implantatsmaterials wird auch das Hydrogel erwärmt und dessen Quelleigenschaften so weit reduziert, dass das Hydrogel eine Formänderung durchläuft, hin zu einem reduzierten Hydrogel 360, welches die Perforationsöffnung in der Membran 320 soweit freigibt, dass Körperflüssigkeiten das biodegradierbare Material 300 des Implantates erreichen können und somit der Korrosionsprozess gestartet wird.

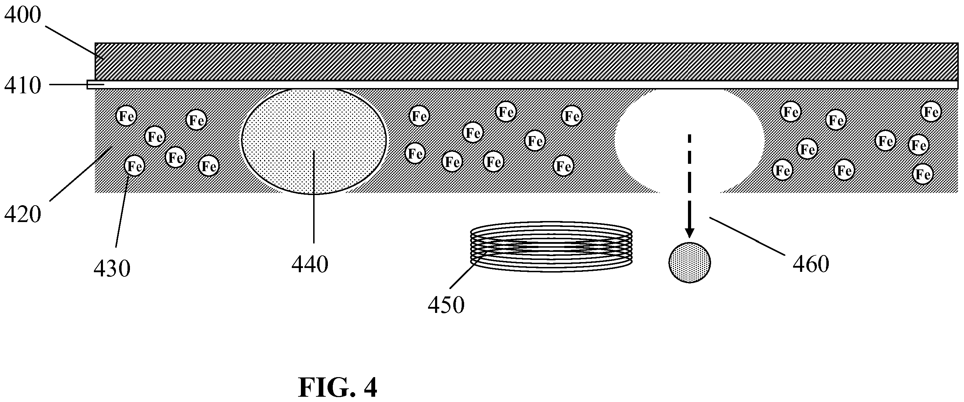

In Figur 4 ist eine alternative Ausführungsform des erfindungsgemäßen Implantates schematisch dargestellt. Hier ist auf dem biodegradierbaren Material des Implantates 400 mittels Haftvermittler 410 eine Schutzschicht bestehend aus einer perforierten Membran 420 aufgebracht. Diese perforierte Membran 420 beinhaltet magnetische Nanopartikel 430. Die Perforationsöffnungen sind auch hier mit einem Hydrogel 440 verschlossen. Bei dieser Ausführungsform kommt ein Hydrogel 440 zum Einsatz mit einem diskontinuierlichen Quellvermögen, welches oberhalb einer bestimmten Temperatur (z. B. 45°-50°C) schlagartig zu einem reduzierten Hydrogel 460 kollabiert und dabei aus der Perforationsöffnung der Schutzschicht "herausfällt". Die Erwärmung der Membran 420 erfolgt hier durch ein mittels eines externen magnetischen Wechselfeldgenerators 450 eingeprägten, wechselnden Magnetfeldes. Dabei werden die magnetischen Nanopartikel 430 in Schwingung versetzt und somit die Membran 420 und das Hydrogel 440 erwärmt.

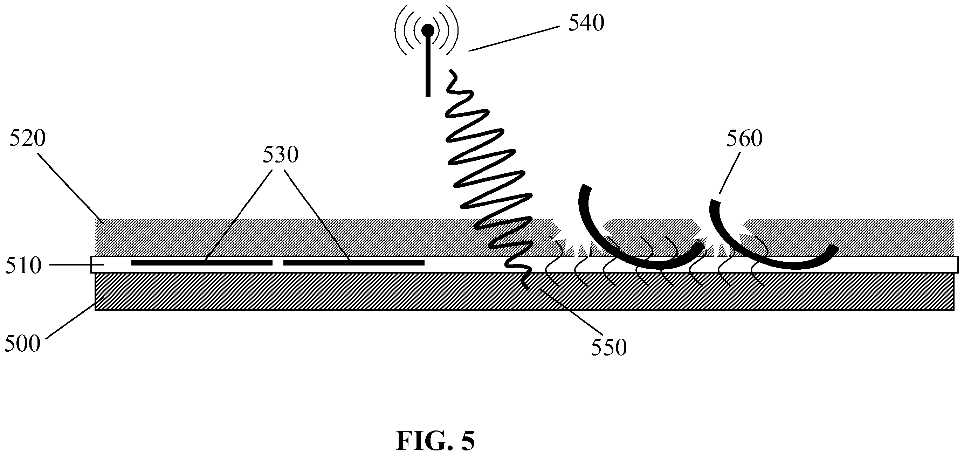

In Figur 5 ist eine weitere Ausführungsform des erfindungsgemäßen Implantates schematisch dargestellt. Hier ist auf dem biodegradierbaren Material des Implantates 500 mittels Haftvermittler 510 eine Schutzschicht bestehend aus einer nicht-perforierten Membran 520 aufgebracht. Der Haftvermittler 510 beinhaltet zusätzlich Formkörper 530 bestehend aus einem biokompatiblen, biodegradierbaren Formgedächtnis-Polymer, wobei die Ursprungsform dieses Formgedächtnis-Polymer eine gebogene Form ist und die gerade, planare Form des Formkörpers 530 den "deformierten Zustand" (eingestellt bei 125°C) des Formgedächtnis-Polymer darstellt. Durch eine mittels eines externen Hochfrequenzsenders 540 eingeprägte Hochfrequenz-Energie werden das biodegradierbare Implantatsmaterial 500 und somit auch Formkörper 530 für mindestens 10 Sekunden auf 45°C erwärmt. Dabei nimmt das Formgedächtnis-Polymer der Formkörper 530 seine ursprüngliche, gebogene Form wieder an. Mittels der nun in der Form veränderten Formkörper 560 wird die Membran 520 perforiert und der Korrosionsprozess gestartet.

Alternativ kann hier die Formveränderung des Formgedächtnis-Polymer durch Licht ausgelöst werden. Eine denkbare Anwendung besteht darin, dass beispielsweise ein Stent angiographisch nachkontrolliert wird und bei einem entsprechenden Befund dessen Degradation durch die direkte Bestrahlung des Stents mittels Lichtleitkatheter mit UV-Licht mit einer Wellenlänge kleine 260 Namometer gestartet wird.

## Patentansprüche

1. Biokorrodierbares Implantat bei dem eine Korrosion nach erfolgter Implantation durch einen externen Stimulus ausgelöst oder beschleunigt werden kann, wobei das Implantat einen Grundkörper (300, 400, 500) aufweist, der ganz oder in Teilen aus einem biokorrodierbaren metallischen Werkstoff besteht, **dadurch gekennzeichnet, dass** der Grundkörper eine Beschichtung mit einer Schutzschicht (320, 420, 520) aufweist, die nicht biokorrodierbar ist, das Implantat Regelelemente (360, 460, 560) aufweist, die derart angeordnet und ausgebildet sind, so dass die Schutzschicht gegebenenfalls in Kombination mit den Regelelementen den Grundkörper ganz oder in Teilen für Körpermedium undurchlässig umschließt, wobei die Schutzschicht durch eine, mittels eines externen Stimulus regel- und/oder steuerbare, Formänderung der Regelelemente in eine für Körpermedium durchlässige Form überführbar ist, und wobei
der externe Stimulus eine Temperaturänderung, elektromagnetische Strahlung, Hochfrequenzstrahlung (RF), ein Ultraschallsignal, ionisierende Strahlung, ein magnetisches Feld und/oder Licht umfasst oder daraus besteht.

2. Implantat nach Anspruch 1, **dadurch gekennzeichnet, dass** der biokorrodierbare Werkstoff eine Magnesiumlegierung ist.

3. Implantat nach einem der vorhergehenden Ansprüche, **dadurch gekennzeichnet, dass** das Implantat eine Gefäßstütze, insbesondere ein Stent, oder ein orthopädisches Implantat ist.

4. Implantat nach einem der vorhergehenden Ansprüche, **dadurch gekennzeichnet, dass** durch den externen Stimulus eine Temperaturänderung im Implantat hervorrufbar ist.

5. Implantat nach einem der vorhergehenden Ansprüche, **dadurch gekennzeichnet, dass** die Schutzschicht magnetische Partikel aufweist.

6. Implantat nach einem der vorhergehenden Ansprüche, **dadurch gekennzeichnet, dass** das Implantat Regelelemente aufweist, bei denen eine Formänderung durch eine Temperaturänderung, elektromagnetischer Strahlung oder UV-Licht regel- und/oder steuerbar ist.

7. Implantat nach einem der vorhergehenden Ansprüche, **dadurch gekennzeichnet, dass** die Schutzschicht eine perforierte Membran umfasst oder daraus besteht und in den Perforationsöffnungen der Schutzschicht Regelelemente angeordnet sind.

8. Implantat nach Anspruch 7, **dadurch gekennzeichnet, dass** die Regelelemente ein Hydrogel enthalten oder daraus bestehen.

9. Implantat nach Anspruch 8, **dadurch gekennzeichnet, dass** das Quellvermögen des Hydrogels temperaturabhängig ist, bevorzugt verringert sich das Quellvermögen bei steigenden Temperaturen.

10. Implantat nach Anspruch 9, **dadurch gekennzeichnet, dass** das Quellvermögen des Hydrogels sich bei einer Temperaturerhöhung um 10K mindestens um 30% verringert.

11. Implantat nach einem der Ansprüche 8 bis 10, **dadurch gekennzeichnet, dass** das Quellvermögen des Hydrogels eine Hysterese aufweist.

12. Implantat nach einem der Ansprüche 1 bis 6, **dadurch gekennzeichnet, dass** die Regelelemente ein Formgedächtnis-Polymer enthalten oder daraus bestehen.

13. Implantat nach einem der Ansprüche 1 bis 6, **dadurch gekennzeichnet, dass** die Schutzschicht als nicht-perforierte, für Körpermedium undurchlässige, Beschichtung vorliegt, und wobei die Regelelemente auf oder in der Schutzschicht angeordnet sind, so dass die Integrität und Dichtigkeit der Schutzschicht gegen Körpermedium durch eine Formänderung der Regelelemente beeinträchtigt und aufgelöst werden kann.

## Claims

1. A biocorrodible implant in which corrosion can be triggered or accelerated by an external stimulus after implantation, the implant having a base body (300, 400, 500) all or parts of which consist(s) of a biocorrodible metal material, **characterized in that** the base body has a coating with a protective layer (320, 420, 520) that is not biocorrodible ,
the implant has control elements (360, 460, 560) that are arranged and shaped in such a way,
that the protective layer, possibly in combination with the control elements, surrounds all or parts of the base body so that it is impermeable to body medium, the protective layer being changeable into a form that it is permeable to body medium by a change in shape of the control elements that is subject to closed-loop or open-loop control by an external stimulus, and
the external stimulus comprising or consisting of a change in temperature, electromagnetic radiation, radio frequency (RF) radiation, an ultrasound signal, ionizing radiation, a magnetic field, and/or light.

2. An implant according to claim 1, **characterized in that** the biocorrodible material is a magnesium alloy.

3. An implant according to any one of the preceding claims, **characterized in that** the implant is a vessel support, especially a stent, or an orthopedic implant.

4. An implant according to any one of the preceding claims, **characterized in that** the external stimulus can cause a change in temperature in the implant.

5. An implant according to any one of the preceding claims, **characterized in that** the protective layer has magnetic particles.

6. An implant according to any one of the preceding claims, **characterized in that** the implant has control elements in which a change in shape is subject to closed-loop or open-loop control by a change in temperature, electromagnetic radiation, or UV light.

7. An implant according to any one of the preceding claims, **characterized in that** the protective layer comprises or consists of a perforated membrane and has control elements arranged in the perforation openings.

8. An implant according to claim 7, **characterized in that** the control elements contain or consist of a hydrogel.

9. An implant according to claim 8, **characterized in that** the swelling capacity of the hydrogel is temperature-dependent, preferably decreasing as the temperature increases.

10. An implant according to claim 9, **characterized in that** the swelling capacity of the hydrogel decreases by at least 30% when the temperature increases by 10K.

11. An implant according to any one of claims 8 through 10, **characterized in that** the swelling capacity of the hydrogel exhibits hysteresis.

12. An implant according to any one of claims 1 through 6, **characterized in that** the control elements contain or consist of a shape memory polymer.

13. An implant according to any one of claims 1 through 6, **characterized in that** the protective layer is in the form of a non-perforated coating that is impermeable to body medium and **in that** the control elements are arranged on or in the protective layer, so that the integrity of the protective layer and its leak-tightness to body medium can be affected by a change in shape of the control elements and eliminated.

## Revendications

1. Implant biocorrodable chez lequel une corrosion peut être déclenchée ou accélérée par un stimulus externe après une implantation réussie, où l'implant présente un corps de base (300, 400, 500) qui est constitué totalement ou en partie d'un matériau métallique biocorrodable, **caractérisé en ce que** le corps de base présente un revêtement avec une couche de protection (320, 420, 520) qui n'est pas biocorrodable,
l'implant présente des éléments de régulation (360, 460, 560) qui sont disposés et conçus de telle manière
**que** la couche de protection, éventuellement en combinaison avec les éléments de régulation, entoure totalement ou en partie le corps de base de manière imperméable pour le milieu corporel, où la couche de protection peut être transformée en une forme perméable au milieu corporel au moyen d'une modification de forme des éléments de régulation commandée par un stimulus externe de régulation et/ou de commande, et où
le stimulus externe comprend une modification de température, un rayonnement électromagnétique, un rayonnement de haute fréquence (HF), un signal ultrasonore, un rayonnement ionisant, un champ magnétique et/ou de la lumière, ou en est constitué.

2. Implant selon la revendication 1, **caractérisé en ce que** le matériau biocorrodable est un alliage de magnésium.

3. Implant selon l'une des revendications précédentes, **caractérisé en ce que** l'implant est une endoprothèse vasculaire, notamment un stent, ou un implant orthopédique.

4. Implant selon l'une des revendications précédentes, **caractérisé en ce qu'**une modification de température dans l'implant peut être induite par le stimulus externe.

5. Implant selon l'une des revendications précédentes, **caractérisé en ce que** la couche de protection présente des particules magnétiques.

6. Implant selon l'une des revendications précédentes, **caractérisé en ce que** l'implant présente des éléments de régulation chez lesquels une modification de forme peut être régulée et/ou commandée par une modification de température, un rayonnement électromagnétique ou par de la lumière UV.

7. Implant selon l'une des revendications précédentes, **caractérisé en ce que** la couche de protection comprend une membrane perforée, ou en est constituée, et que des éléments de régulation sont disposés dans les orifices de perforation de la couche de protection.

8. Implant selon la revendication 7, **caractérisé en ce que** les éléments de régulation contiennent un hydrogel ou en sont constitués.

9. Implant selon la revendication 8, **caractérisé en ce que** le pouvoir de gonflement de l'hydrogel est dépendant de la température, de préférence, le pouvoir de gonflement diminue pour des températures croissantes.

10. Implant selon la revendication 9, **caractérisé en ce que** le pouvoir de gonflement de l'hydrogel diminue d'au moins 30 % avec une croissance de la température d'environ 10 K.

11. Implant selon l'une des revendications 8 à 10, **caractérisé en ce que** le pouvoir de gonflement de l'hydrogel présente une hystérèse.

12. Implant selon l'une des revendications 1 à 6, **caractérisé en ce que** les éléments de régulation contiennent un polymère à mémoire de forme ou en sont constitués.

13. Implant selon l'une des revendications 1 à 6, **caractérisé en ce que** la couche de protection se présente sous la forme d'un revêtement non perforé, imperméable au milieu corporel et où les éléments de régulation sont disposés sur ou dans la couche de protection de sorte que l'intégrité et l'étanchéité de la couche de protection vis-àvis du milieu corporel peut être influencée ou désagrégée par une modification de forme des éléments de régulation.
